# EUROPEAN PATENT SPECIFICATION

(11) **EP 0 571 111 B1**
(45) Date of publication and mention of the grant of the patent: **13.08.1997**
(21) Application number: 93303575.0
(22) Date of filing: 07.05.1993
(51) Int. Cl.: B29C 61/04, B29C 65/66

(54) **Tubular assembly**
Zusammengestelltes Rohr
Assemblage tubulaire

(30) Priority: 22.05.1992 US 887993
(43) Date of publication of application: 24.11.1993
(73) Proprietor: Diametrics Medical Ltd., High Wycombe, Buckinghamshire PH13 5RE (GB)
(72) Inventor: Markle, David R., Paoli, Pennsylvania 19301 (US); Paterson, William, High Wycombe, Buckinghamshire PH13 5RE (GB)
(74) Representative: Woodcraft, David Charles

(56) References cited:
- EP-A- 0 439 284
- DE-U- 9 010 609
- FR-A- 2 215 123
- GB-A- 2 195 069
- US-A- 3 861 972
- US-A- 4 178 067

## Description

This invention relates to a tubular assembly, a method of assembly, of a hollow elongated tube and an apparatus for assembling a tube and a plug according to the respective precharacterising parts of claims 1, 6 and 8. More particularly, the invention is concerned with a tubular assembly comprising a tube and a plug located at least partially within the tube. The plug is preferably made of a thermoplastic polymer and relaxation of residual stresses or release of the memory of the manufacturing process used to make the part allows expansion of the plug so that it engages the inside surface of the tube.

Typically tubes are stoppered, plugged or sealed by application of a press fit, shrink fit or wedging component into the bore thereof. Chemicals such as adhesives, sealants or glues are frequently applied to make a fluid tight joint. The accurate application of chemicals to make a small joint fluid tight presents added difficulties of placement and clean up. Often the chemicals used may interfere with the operation of the assembly being made therewith, and also chemical may leach into the blood stream during use of a tubular probe.

Optical fibers or fiber optic chemical sensors used in vivo as probes must be sensitive to slight changes in gas or ion concentrations. For example, U.S. patent No. 4,200,110 discloses a fiber optic pH probe comprising an ion permeable membrane envelope about the distal ends of a pair of optical fibers. The probe operates on the concept of optically detecting the change in color of a pH sensitive dye.

U.S. Patent Reissue 31,879 discloses a method for measuring concentration of an analyte in a sample by measuring the intensity of light emitted from a fluorescent indicator attached to an optical fiber.

U.S. Patent No. 5.047,208 discloses a calorimetric fiber optic sensor for blood gas measurement comprising a pH sensitive dye in a chamber at the distal end of an optical fiber. A white reflective surface is located distal to the chamber.

The above patents disclose typical miniaturized structures required for in vivo blood analysis.

U.S. Patent No. 5,005,576 discloses an optical probe for invasive blood gas measurement comprising sensors capable of measuring pH, pO₂ and pCO₂ using absorption dyes. A reflector unit is positioned at the distal end of an outer sheath having a gas-permeable adhesive to close off the end of the probe.

U.S. Patent No. 5,047,627 discloses a multi-analyte sensor comprising three optical fibers each associated with an indicator matrix and a light reflectance material. Certainty and consistency of assembly is not always repeatable with adhesive attachment, and chemicals in the adhesive may adversely influence the chemistry of the sensor and therefor measurement accuracy.

U.S. Patent No. 4,889,407 discloses an optical waveguide sensor having a plurality of cells arranged in an array which substantially covers the cross sectional area of the waveguide. Each of the cells contains an indicator sensitive to an analyte in a medium, particularly for the determination of pH and pCO₂ in vivo in blood,

French patent application no FR-A-2215123 discloses a thermoplastic material which expands on heating and retains the expanded form after cooling. The tubular material is used for joining two tubes together or for sealing a tube in a frame.

EP-A-0439284 discloses a cable joint closure including a heat expandable inner member while GB-A-2195069 discloses an electric heater for shrinking sleeves about cable splices.

It is an object of the present invention to provide a simple means of mounting a plug within a tubular assembly in order to form a fluid tight seal. The method is of particular value in the construction of blood monitoring probes and catheters.

In accordance with the present invention there is provided a tubular assembly comprising a hollow elongated tube having an internal cross section with an inside surface, a proximal end and a distal end defining a bore around a longitudinal axis, and a plug mounted within the bore so that it is at least partially within the tube, characterised by the plug having been thermally treated to expand and engage the inside surface of the tube so that, after change in temperature, the plug has an external cross sectional size identical to the internal cross sectional size of the tube and thereby forms a fluid tight seal which seals the distal end of the tube.

Particular embodiments of the invention according to claim 1 are subject of the dependent claims 2 to 5.

The tube preferably has an internal cross sectional shape identical to the plug and a cross sectional shape identical to the plug and a cross sectional size larger than the plug prior to changing temperature. The plug has an external cross sectional size identical to the internal cross sectional size of the tube after change in temperature. Preferably, the tube has a substantially circular internal cross sectional shape defined by the bore through the tube and the plug has a substantially circular external cross sectional shape of a diameter that allows axial movement within the tube before thermal treatment.

The plug should preferably have a cross sectional shape and size to be near the inside surface upon placement of the part at least partially within the tube. The plug or the tube may be supported so that relative movement along the axis places the plug at least partially within the tube. The plug is preferably made of a thermoplastic polymer by a process which develops within the polymer a memory of the unshaped thermoplastic polymer prior to forming into the cross sectional shape of the plug so that residual stresses remaining in the formed plug are relaxed by changing temperature causing expansion of the cross sectional size of the tube and/or the plug. The plug and tube are preferably made by an extrusion process to develop memory. The plug may be located in the tube between the ends thereof or located in the tube near the proximal or distal end thereof.

A form, preferably made of a relatively nonconductive material, is preferably positioned for relative movement along the axis for placement about the tube whereinside the plug is located. The form is preferably shaped and sized to fit the tube outside surface and contain it. The form, the tube and the plug are preferably substantially circular in cross section.

A heater, preferably made of an electrically resistive element, is wrapped about the form and is associated therewith for changing the temperature of the tube and the plug sufficiently to relax residual stresses of their respective manufacturing processes, and thereby cause engagement of the inside surface of the tube and the plug. The heater changes the temperature beyond the glass transition point of the polymer allowing the combined tube and plug to expand to the identical cross sectional size of the internal cross section of the form and to remain expanded thereafter. As used herein, engagement means that if the plug and tube are made of materials that are heat sealable with respect to one another, then they will fuse together when they are thermally heated. Alternatively, if the materials will not fuse then they form a tight fit therebetween upon engagement.

The invention also provides a method of assembly of a hollow elongated tube with an axis along the longitude thereof and with inside and outside surfaces, a proximal end and a distal end and a plug with a cross sectional shape and size similar to the inside surface of the tube when the plug is at least partially within the tube using a form shaped and sized to fit about the outside surface of the tube and a heater associated with the form, which comprises the steps of supporting the tube and the plug so that relative movement along the tube axis places the plug at least partially within the tube; positioning the form for relative movement along the axis for placement about the tube whereinside the plug is located to thereby contain the outside surface of the tube; subjecting the tube and the plug to thermal treatment to change the temperature sufficiently to relax residual stresses and thereby cause engagement of the inside surface of the tube and the plug which engagement forms a fluid-tight seal which seals the distal end of the tube.

Particular embodiments of the invention according to claim 6 are subject of the dependent claim 7.

The method preferably also includes the added steps of clearing fluid about the junction between the plug and the tube before heating. Preferably sufficient heat is conducted through the form into the plug to form a seal between the plug and the inside surface of the tube. The step of clearing fluid is performed most preferably by applying a vacuum near the plug during placement.

The invention also provides an apparatus for assembling a tube and a plug inserted at least partially therewithin, characterized by a hollow elongated tube with an axis along the longitude thereof, and with inside and outside surfaces, a proximal end and a distal end; a plug with a cross sectional shape and size similar to the inside surface when the plug is at least partially within the tube, the plug and the tube being supported so that relative movement along the axis places the plug at least partially within the tube; a form positioned for relative movement along the axis for placement about the tube whereinside the plug is located, the form shaped and sized to fit the tube outside surface and contain it; and a heater associated with the form for providing thermal treatment to change the temperature of the tube and the plug sufficiently to relax residual stresses and thereby permit engagement between the inside surface of the tube and the plug, which engagement forms a fluid-tight seal which seals the distal end of the tube.

Particular embodiments of the invention according to claim 8 are subject of the dependent claims 9 and 10.

The invention will be particularly described with reference to preferred embodiments illustrated in the accompanying drawings in which:-
Figure 1 is a side view in cross section of a distal end of a tube with the plug located at least partially therewithin for sealing the distal end of the tube; the form is about the tube whereinside the plug is located;
Figure 2 is a side view in cross section of a plurality of sensors extending through a radially expanded section positioned within a tube to illustrate sealing to the tube and the sensors as the first step in the assembly of a sensor catheter;
Figure 3 is a side view in cross section of the sealed sensors in a radially expanded section of a tube wherein the distal end thereof is sealed with a plug while a vacuum is drawn to clear the area around the sensors;
Figure 4 is a side view in cross section of an optical fiber extending through a tube to illustrate sealing between the tube and the fiber as a step in the assembly of a sensor catheter;
Figure 5 is a side view in cross section of the sealed fiber, radially expanded within the tube wherein the fiber is engaged tightly against the inside of the tube;
Figure 6 is a side view in cross section of the sealed fiber in a radially expanded section of a tube, wherein the distal end thereof is sealed with a plug while a vacuum draws liquid displaced when the plug is pushed into the distal end of the tube; and
Figure 7 is a side view in cross section of the fiber and plug sealed in the tube with the space therebetween filled completely with liquid.

The most preferred application of the tubular assembly of the present invention is for the construction of a small (less than 1 mm (20 gauge)) blood gas sensor catheter. Specifically the assembly involves sealing of the distal portion of blood gas sensors within a vascular catheter sheath about sensors such as electrical wires and/or optical fibers passing axially therethrough and termination of the leading end of the blood gas catheter.

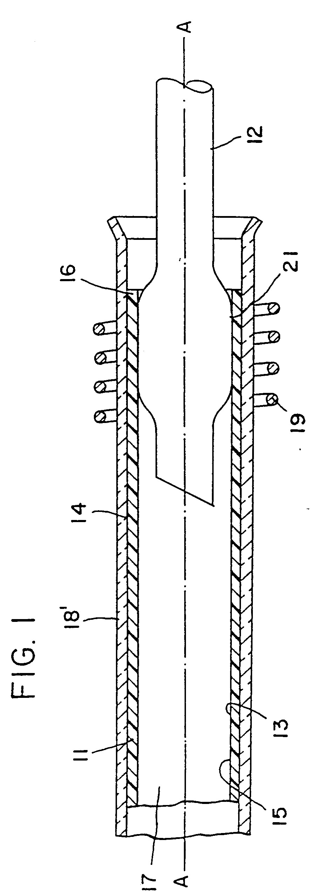

In Figure 1 of the drawings the tubes 11 has as inside surface 13 and an outside surface 14 extending between a proximal end 15 and a distal end 16. In a preferred embodiment the tube 11 is used as a sheath for a blood gas sensor accommodated in a vascular catheter and has an internal cross sectional shape identical to that of the plug 12 and a cross sectional size slightly larger than that of the plug 12 prior to being subjected to thermal treatment, as described hereinafter. The plug 12 may take various forms, but it is preferred that the plug 12 have an external cross sectional size substantially identical to the internal cross sectional size of the tube 11 after thermal treatment so that effective sealing is achieved.

In the preferred embodiment, i.e. a blood gas sensor for a vascular catheter, the tube 11 has an internal circular cross sectional shape defined by a bore 17 through the tube 11. Similarly, the plug 12 has a circular cross sectional shape of a diameter that may allow axial movement within the tube 11 prior to sealing. The plug 12 preferably should have a cross sectional shape and size to be substantially near the inside surface 13 upon placement of the plug 12 at least partially within the tube 11. Preferably the plug 12, if used to seal the distal end 16, should be slightly tapered to displace any liquid in the tube 11 so that a bubble-free fill remains when the plug 12 and tube 11 are sealed.

An apparatus for assembly of the tube 11 and the expanded plug 12 supports the plugs 12 and/or the tube 11 for relative movement along the axis A placing the plug 12 at least partially within the tube 11. The precision and speed necessary to place the plug 12 within the tube 11 is easily accomplished by automatic machinery that aligns the plug 12 and the tube 11 with the axis A so that each is centered and may be placed concentric to one another.

Figure 1 is a side view in cross section of the form 18' used for assembly of the tube 11 and the plug 12 and wherein the plug 12 is now located at least partially within the distal end 16 of the tube 11. The plug 12 is inserted into the distal end 16 not through the tube 11 although that is a possible option. Preferably each of the plug 12 and/or the tube 11 is made of a thermoplastic polymer by a process that develops within the polymer a memory of the unshaped thermopastic polymer prior to forming into the cross sectional shape of the plug 12 and/or the tube 11. While the plug 12 and the tube 11 may be made from the same polymer they also may be made from different polymers. When the same polymer is used the sealing includes melting and when different polymers are used the sealing is a result of expansion which forms a tight interference fit.

Residual stresses remaining in the formed plug 12 and/or tube 11 may be relaxed by changing temperature causing expansion of the cross sectional size of the tube 11 and/or the plug 12. The plug 12 and/or the tube 11 are preferably made by an extrusion process to develop such processing memory. The changing cross sectional size of the tube 11 may provide a thicker wall section after heating and the plug 12 may swell when heated. During the heating process the tubes 11, as necessary, is urged axially toward the area of heating to prevent thinning which could result in an opening or tear in the tube 11. The residual stress in the memory of the process used to make the tube 11, i.e. extrusion, could cause the tube 11 to pull apart. Urging the tube 11 is accomplished by feeding the tube 11 axially toward the area of heating from one direction and the plug 12, such as the monofilament, from the other toward the middle with sufficient force to prevent thinning. The fused joint (sane polymer) between the plug 12 and the tube 11 is therefore uniformly around the plug 12 and the seal is sufficient to plug the distal end 16 of the tube 11.

The form 18' preferably made of a relatively nonconductive material such as tempered glass and the form (18') is preferably positioned for relative movement along the axis A for placement about the plug 12 and may be placed over the tube 11 whereinside the plug 12 is located as shown in Figure 1. The form 18' is shaped and sized to fit the tube 11 inside surface 13 and outside surface 14, respectively and contain it. The form 18', the tube 11 and the plug 12 are preferably substantially circular in cross section. The dimensions are not critical but should be selected to provide the type of fit desired between form 18', the plug 12 and the tube 11. That is, a slip fit during initial assembly so that the tube 11 fits easily into the form 18' and the plug 12, which is preferably tapered, fits into the form 18' and thereafter the tube 11 to displace liquid. The drawings show clearance between the plug 12 and the tube 11 but that is for illustration only since the fit is not loose.

A heater 19 is preferably made of an electrically resistive element wrapped about an outer wall 20 of the form 18' and is associated therewith for changing the temperature of the tube 11 and the plug 12 therewithin sufficiently to relax residual stresses resulting from their respective manufacturing processes. The heat applied will thereby cause expansion and engagement of the inside surface 13 and the plug 12. The heater 19 raises the temperature beyond the glass transition point of the polymer of the tube 11 and/or the plug 12 allowing the combined tube 11 and plug 12 to expand to the identical cross sectional size of the internal cross section of the form 18' and to remain expanded after cooling. Melting of the polymer is not required, unless the same polymers are used. Otherwise, sealing is sufficiently accomplished when expansion takes place while the plug 12 and the tube 11 are contained by the form 18'. Thus the contained expansion is adequate to create a joint 21 which will perform acceptably in connection with blood gas sensors. Expansion is for dissimilar polymers and fusion in the form of a melted heat seal occurs between similar polymers.

The preferred method of assembly of the tube 11 with its axis along its longitude and inside and outside surfaces 13 and 14 extending between proximal and distal ends 15 and 16 over plug 12 of a cross sectional shape and size to be near the inside surface 13 of the tube when the plug 12 is at least partially within the tube 11 has several steps. The method may be performed as shown in the drawings with parts of various configurations. Form 18' is shaped and sized to fit about the tube 11 outside surface 14 with heater 19 about outer wall 20 of the form 18'. The next step comprises supporting the tube 11 and the plug 12 so that relative movement therebetween and along axis A places the plug 12 at least partially within the tube 11. That step is followed by positioning form 18' for relative movement along axis A for placement about the tube 11 whereinside the plug 12 is located to thereby contain the outside surface. 14. Sufficiently changing the temperature of the tube 11 and the plug 12 relaxes residual stresses resulting from the manufacturing process used to make the tube 11 and/or the plug 12 for causing engagement of the inside surface 13 of the tube 11 and the plug 12.

The method preferably also includes the added steps of conducting sufficient heat through form 18' into the tube 11 and/or the plug 12 to relax residual stresses therein with or without melting the tube 11 and/or the plug 12 and clearing fluid (or liquid) about the plug 12 before heating. The step of clearing fluid is preferably performed by moving a gas along the tube 11 and the step of moving is most preferably performed by applying a vacuum to the junction between tube 11 and plug 12.

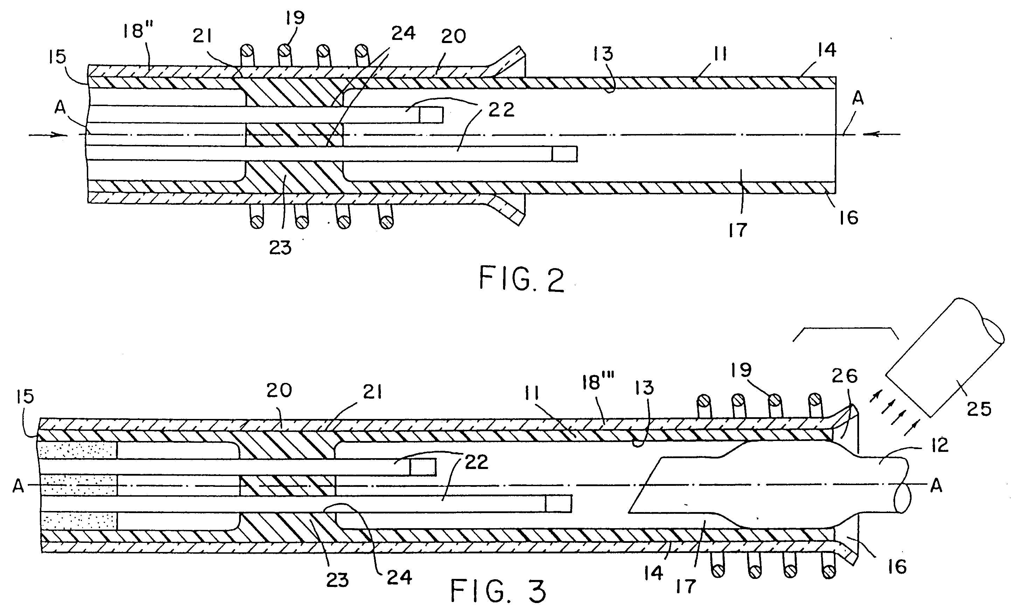

Figure 2 is a side view in cross section of a plurality of sensors 22 extending through a radially inwardly expanded section 23 located within the tube 11 to illustrate supporting the sensors 22 with the tube 11 as the first step in the assembly of a sensor catheter. The radially inwardly expanded section 23 acts to support and separate the sensors of a blood gas catheter sensor. The radially inwardly protruding section 23 in Figure 2 shows two sensors passing through respective passageways 24 formed within the tube 11. The heater 19 surrounds a form 18'' and heat applied therewith changes the temperature of the tube 11 creating the radially inwardly expanded section 23 within the tube 11 after overcoming the relaxation of residual stresses within the polymer of the tube 11 by pushing the tube axially thus feeding sufficient polymer into the radially inwardly expanding section 23. The result is that the radially inwardly protruding section 23 swells when the tube 11 expands and the sensors 22 are captured, supported and held separately in passages 24. The preferred embodiment includes temperature sensors made of silver, platinum or gold wire, which are all good conductors of heat. Therefore, to heat properly and prevent the loss of heat to the wires, heating is conducted at just below the melting temperature of the tube 11 polymer, for example, polyethylene, and then quickly for a short time with a pulse of energy through the heating wire coil the temperature is raised to melt the tube 11.

A seal is thus provided between the tube 11, the radially inwardly protruding section 23 and the sensors 22. During the heating process the tube 11, as necessary, is urged axially toward the area of heating to provide thickening at the radially inwardly protruding section 23. Therefore an opening or tear in the tube 11 is prevented with the added material provided by feeding tube 11 into the space between the sensors 22 and the inside of the tube 11. Urging the tube 11 is preferably accomplished by feeding the tube 11 axially toward the area of heating from either direction; that is, each end 15 and 16 is pushed toward the middle with sufficient force to provide thickening around the sensors 22. The joint between the sensors and the tube 11 is therefore uniform around the sensors 22 and the seal is accomplished without added cement.

An assembly, shown in Figures 2 and 3, is easily realized even though the dimensions of the components of the blood gas sensor catheter are small and typically miniaturized. More significantly, the chemistry of the sensor used for optically or electrochemically sensing blood gases may be sensitive to adhesives and so deletion or isolation is of benefit for this reason.

In Figure 3, a side view in cross section of the supported sensors 22 held within the radially inwardly expanded section 23 of the tube 11 illustrates how the distal end 16 thereof is plugged with the plug 12 while a vacuum is drawn to clear liquid resulting when the plug 12 is plugged into the tube 11. A pipe 25 is located near the distal end (16), i.e. just outside but in position to draw a vacuum near a junction 26 between the plug 12 and the tube 11 are heated during the assembly process. Arrows B illustrate the vacuum in Figure 3. It should be appreciated that, although not preferred, a sealant may be drawn into the tube 11 to fill the proximal end 15 and that is shown by the dark areas between the sensors 22 on the left side of Figure 3. While not specifically shown, the plug 12 after being sealed in the distal end 16 as in Figures 1 and 3 may be cut to provide a desired shape at the sealed distal end 16. The distal seal is accomplished by first pushing the tapered plug into the tube 11 distal end 16 thus displacing liquid therein and drawing excess liquid away with vacuum. Then heating to form a fluid-tight seal between the plug 12 and tube 11. Thus, a completely liquid filled space devoid of air bubbles is attained.

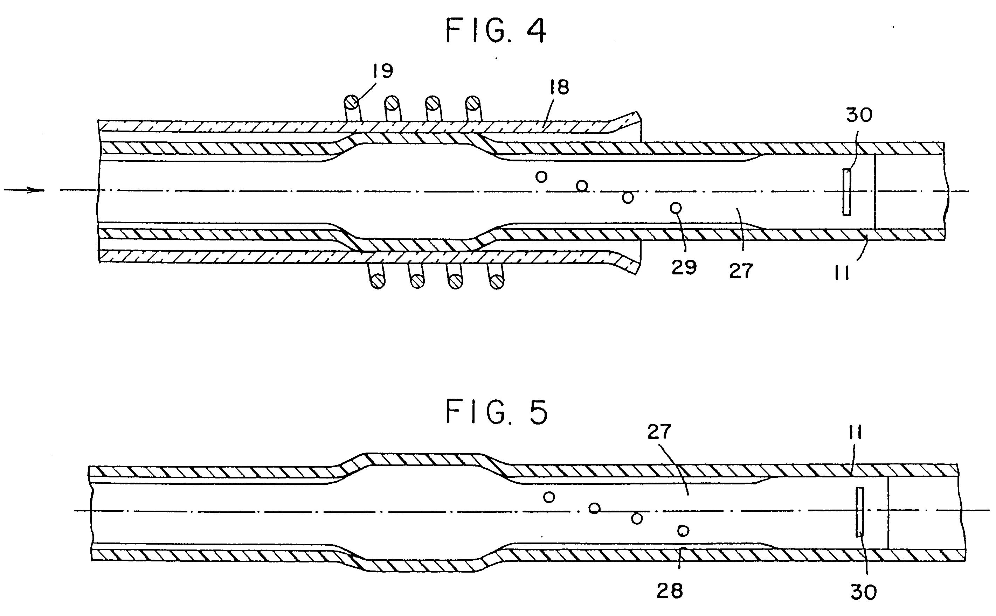

In Figure 4, an optical fiber sensor 27 prepared in accordance with the disclosure in U.S. Patent No. 4,889,407 is shown positioned coaxially within the tube 11 and expanded to engage the inside of the tube 11 by the heater as described hereinbefore. The sensor comprises cells 29 arranged in a helical array which substantially covers the cross-sectional area of the fiber and a reflective mirror 30 embedded near the distal end of the optical fiber in accordance with the disclosure in U.S. Patent No. 5257338. The fiber 27 and the tube 11 are shown being sealed together in the side view in cross section of Figure 5. An annular space 28 surrounds a distal portion of the fiber 27.

Figure 5 is a side view in cross section of the sealed fiber 27 and tube 11 shown removed from the glass form used to restrict radial expansion during the heating. During the heating process the tube 11, as necessary, is urged axially toward the area of heating to prevent thinning which could result in an opening or tear in the tube. Urging the tube 11 is preferably accomplished by feeding the tube 11 axially toward the area of heating from either direction; that is, each end is pushed toward the middle with sufficient force to prevent thinning. The joint between the fiber 27 and the tube 11 is therefore uniform around the fiber 27 and the seal is sufficient to maintain a liquid in the annular space 28 as needed.

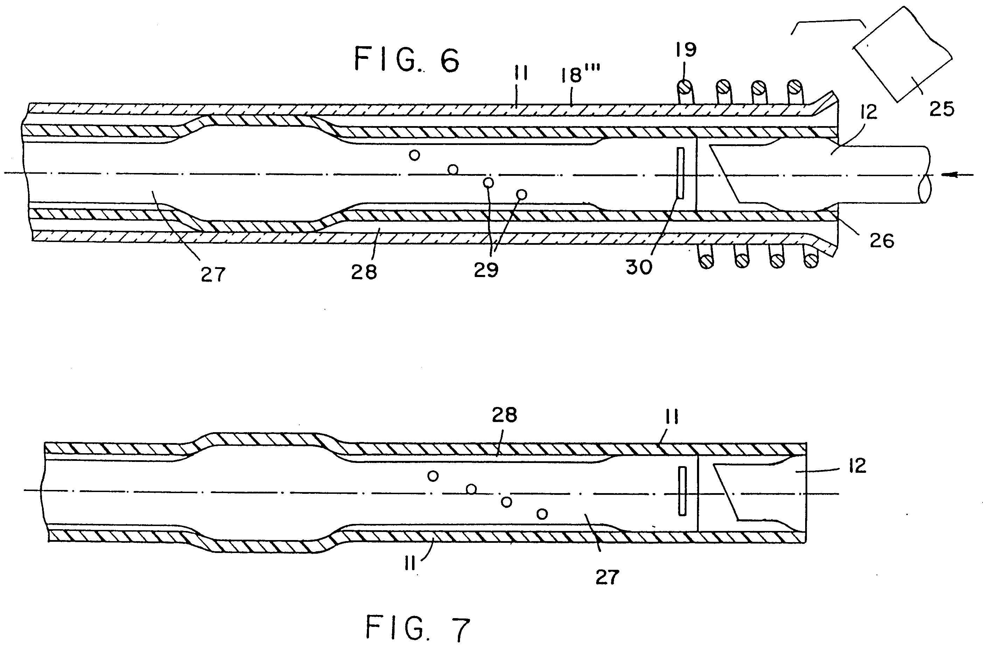

Figures 6 and 7 show a tip sealing process and apparatus similar to that disclosed in connection with Figures 1, 2 and 3. In these Figures 6 and 7 the tube 11 has a monofilament as plug 12 and the plug to seal the end.

## Claims

1. A tubular assembly comprising a hollow elongated tube(11) having an internal cross section with an inside surface(13), a proximal end and a distal end(16) defining a bore around a longitudinal axis, and a plug(12) mounted within the bore so that it is at least partially within the tube(11) characterized by the plug(12) having been thermally treated to expand and engage the inside surface of the tube(11) so that after change in temperature, the plug(12) has an external cross sectional size identical to the internal cross sectional size of the tube(11), and thereby forms a fluid tight seal which seals the distal end(15) of the tube.

2. A tubular assembly according to claim 1, characterized in that the internal cross section of the tube(11) and the external cross section of the plug(12) which engages the inside surface of the tube is substantially circular.

3. A tubular assembly according to claim 1 or 2 characterized in that the plug(12) is made of a thermoplastic polymer.

4. A tubular assembly according to any one of claims 1 to 3, characterized in that the tube(11) accommodates one or more sensors mounted parallel to the longitudinal axis.

5. A tubular assembly according to any one of claims 1 to 4, characterized in that the tube(11) is made from an extruded thermoplastic polymer and the thermal treatment thereof results in relaxation of residual stresses and causes the inside surface thereof to move toward the plug(12) for engagement.

6. A method of assembly of a hollow elongated tube(11) with an axis along the longitude thereof and outside surfaces, a proximal end and a distal end(16) and a plug(12) with a cross sectional shape and size similar to the inside surface of the tube(11) when the plug(12) is at least partially within the tube(11) using a form(18') shaped and sized to fit about the outside surface of the tube(11) and a heater(19) associated with the form(18'); characterized by the following steps:
supporting the tube(11) and the plug(12) so that relative movement therebetween and along the tube(11) axis places the plug(12) at least partially within the tube(11);
positioning the form(18') for relative movement along the axis for placement about the tube(11) whereinside the plug(12) is located to thereby contain the outside surface of the tube;
subjecting the tube(11) and the plug(12) to thermal treatment to change the temperature sufficiently to relax residual stresses and thereby cause engagement of the inside surface of the tube(11) and the plug(12), which engagement forms a fluid-tight seal which seals the distal end(16) of the tube(11).

7. A method according to claim 6, including the step of clearing fluid about the junction between the plug (12) and the tube(11) before heating.

8. An apparatus(10) for assembling a tube(11) and a plug(12) inserted at least partially therewithin, characterized by a hollow elongated tube(11) with an axis along the longitude thereof, and with inside and outside surfaces, a proximal end and a distal end(16);
a plug(12) with a cross sectional shape and size similar to the inside surface when the plug(12) is at least partially within the tube(11), and the plug(12) and tube(11) being supported so that relative movement along the axis places the plug(12) at least partially within the tube(11);
a form(18') positioned for relative movement along the axis for placement about the tube(11) whereinside the plug(12) is located, the form shaped and sized to fit the tube(11) outside surface and contain it, and
a heater(19) associated with the form for providing thermal treatment to change the temperature of the tube(11) and the plug(12) sufficiently to relax residual stresses and thereby permit engagement between the inside surface of the tube(11) and the plug(12), which engagement forms a fluid-tight seal which seals the distal end(16) of the tube(11).

9. An apparatus according to claim 8, characterized in that the tube(11) has a substantially circular internal cross section and a cross sectional size larger than the plug(12) prior to thermal treatment and the plug has a substantially circular external cross section identical in size to the internal cross section of the tube(11) after thermal treatment.

10. An apparatus according to claim 8 or 9, characterized in that the plug(12) is made of a thermoplastic polymer by a process that develops within the polymer a memory of the unshaped thermoplastic polymer prior to forming into the cross sectional shape of the plug(12) so that residual stresses remaining in the plug(12) are relaxed by changing temperature causing expansion of the cross sectional size of the plug(12).

## Patentansprüche

1. Rohrartiger Aufbau, umfassend ein hohes langgestrecktes Rohr (11), welches einen Innenquerschnitt mit einer Innenoberfläche (13), einem proximalen Ende und einem distalen Ende (16), der eine Öffnung um eine Längsachse herum definiert, und einen Stopfen (12) aufweist, welcher innerhalb der Öffnung derart angebracht ist, daß er wenigstens teilweise innerhalb des Rohrs (11) ist, dadurch gekennzeichnet, daß der Stopfen (12) thermisch behandelt worden ist, um sich zu erweitern und an der Innenoberfläche des Rohrs (11) anzugreifen, so daß nach einer Temperaturänderung der Stopfen (12) eine Außenquerschnittsgröße aufweist, die gleich der Innenquerschnittsgröße des Rohrs (11) ist, und dadurch eine fluiddichte Abdichtung bildet, welche das distale Ende (15) des Rohrs abdichtet.

2. Rohrartiger Aufbau nach Anspruch 1, dadurch gekennzeichnet, daß der Innenquerschnitt des Rohrs (11) und der Außenquerschnitt des Stopfens (12), welcher an der Innenoberfläche des Rohrs angreift, im wesentlichen kreisförmig sind.

3. Rohrartiger Aufbau nach Anspruch 1 oder 2, dadurch gekennzeichnet, daß der Stopfen (12) aus einem thermoplastischen Polymer gebildet ist.

4. Rohrartiger Aufbau nach einem der Ansprüche 1 bis 3, dadurch gekennzeichnet, daß das Rohr (11) einen oder mehrere parallel zu der Längsachse angebrachte Sensoren aufnimmt.

5. Rohrartiger Aufbau nach einem der Ansprüche 1 bis 4, dadurch gekennzeichnet, daß das Rohr (11) aus einem extrudierten thermoplastischen Polymer gebildet ist und daß die thermische Behandlung desselben zu einer Relaxation von Restspannungen führt und verursacht, daß sich die Innenoberfläche desselben zum Angreifen auf den Stopfen (12) zu bewegt.

6. Verfahren zum Zusammensetzen eines hohen langgestreckten Rohrs (11) mit einer Achse entlang der Länge desselben und Außenoberflächen, einem proximalen Ende und einem distalen Ende (16) und eines Stopfens (12) mit einer Querschnittsform und Größe, welche der Innenoberfläche des Rohrs (11) entsprechen, wenn der Stopfen (12) wenigstens teilweise in dem Rohr (11) ist, unter Verwendung einer Form (18'), welche derart geformt und bemessen ist, daß sie um die Außenoberfläche des Rohrs (11) paßt, und eines Heizers (19), welcher der Form (18') zugeordnet ist, gekennzeichnet durch die folgenden Schritte:
- Tragen der Rohrs (11) und des Stopfens (12) derart, daß eine Relativbewegung zwischen diesen und entlang der Achse des Rohrs (11) den Stopfen (12) wenigstens teilweise innerhalb des Rohrs (11) plaziert,
- Positionieren der Form (18') zur Relativbewegung entlang der Achse zum Plazieren um das Rohr (11) herum, innerhalb welchem der Stopfen (12) angeordnet ist, um dadurch die Außenoberfläche des Rohrs aufzunehmen,
- Aussetzen des Rohrs (11) und des Stopfens (12) einer thermischen Behandlung zum ausreichenden Ändern der Temperatur, um Restspannungen zu relaxieren und dadurch ein Aneinanderangreifen der Innenoberfläche des Rohrs (11) und des Stopfens (12) zu bewirken, welches Aneinanderangreifen eine fluiddichte Dichtung bildet, die das distale Ende (16) des Rohrs (11) abdichtet.

7. Verfahren nach Anspruch 6, umfassend den Schritt des Entfernens von Fluid um die Verbindung zwischem dem Stopfen (12) und dem Rohr (11) vor dem Heizen.

8. Einrichtung (10) zum Zusammensetzen eines Rohrs (11) und eines Stopfens (12), welcher wenigstens teilweise in dieses eingeführt ist, gekennzeichnet durch ein hohles, langgstrecktes Rohr (11) mit einer Achse entlang der Länge desselben und mit Innen- und Außenoberflächen, einem proximalen Ende und einem distalen Ende (16),
einen Stopfen (12) mit einer Querschnittsform und Größe, welche der Innenoberfläche entsprechen, wenn der Stopfen wenigstens teilweise innerhalb des Rohrs (11) ist, und wobei der Stopfen (12) und das Rohr (11) derart getragen sind, daß eine Relativbewegung entlang der Achse den Stopfen (12) wenigstens teilweise innerhalb des Rohrs (11) plaziert,
- eine Form (18'), welche zur Relativbewegung entlang der Achse zum Plazieren um das Rohr (11) herum positioniert ist, innerhalb welchem der Stopfen (12) angeordnet ist, wobei die Form derart geformt und bemessen ist, daß sie auf die Außenoberfläche des Rohrs (11) paßt und dieses aufnimmt, und
einen Heizer (19), welcher der Form zugeordnet ist zum Vorsehen einer thermischen Behandlung und zum ausreichenden Ändern der Temparatur des Rohrs (11) und des Stopfens (12), um Restspannungen zu relaxieren und dadurch ein Aneinanderangreifen der Innenoberfläche des Rohrs (11) und des Stopfens (12) zu ermöglichen, welches Aneinanderangreifen eine fluiddichte Abdichtung bildet, die das distale Ende (16) des Rohrs (11) abdichtet.

9. Einrichtung nach Anspruch 8, dadurch gekennzeichnet, daß das Rohr (11) einen im wesentlichen kreisförmigen Innenquerschnitt und eine Querschnittsgröße aufweist, die größer ist als der Stopfen (12) vor der thermischen Behandlung, und daß der Stopfen einen im wesentlichen kreisförmigen Außenquerschnitt aufweist, der in seiner Größe gleich dem Innenquerschnitt des Rohrs (11) nach der thermischen Behandlung ist.

10. Einrichtung nach Anspruch 8 oder 9, dadurch gekennzeichnet, daß der Stopfen (12) aus einem thermoplastischen Polymer durch ein Verfahren gebildet ist, welches in dem Polymer ein Formgedächtnis des nicht geformten thermoplastischen Polymers vor dem Formen in die Querschnittsform des Stopfens (12) entwickelt, so daß in dem Stopfen (12) verbleibende Restspannungen durch Temperaturänderung relaxiert werden, wodurch eine Aufweitung der Querschnittsgröße des Stopfens (12) verursacht wird.

## Revendications

1. Assemblage tubulaire comprenant un tube creux allongé (11) ayant une section transversale intérieure avec une surface intérieure (13), une extrémité proximale et une extrémité distale (16) définissant un perçage autour d'un axe longitudinal, et un bouchon (12) monté à l'intérieur du perçage de sorte qu'il se trouve au moins partiellement à l'intérieur du tube (11), caractérisé en ce que le bouchon (12) est traité thermiquement pour se dilater et se lier à la surface intérieure du tube (11) de sorte qu'après modification de température, le bouchon (12) possède une dimension extérieure en section transversale identique à la dimension intérieure en section transversale du tube (11), et de façon à former un joint étanche au fluide qui ferme hermétiquement l'extrémité distale (15) du tube.

2. Assemblage tubulaire selon la revendication 1, caractérisé en ce que la section transversale intérieure du tube (11) et la section transversale extérieure du bouchon (12) qui se lie avec la surface intérieure du tube sont sensiblement circulaires.

3. Assemblage tubulaire selon la revendication 1 ou 2, caractérisé en ce que le bouchon (12) est réalisé en un polymère thermoplastique.

4. Assemblage tubulaire selon l'une quelconque des revendications 1 à 3, caractérisé en ce que le tube (11) reçoit un ou plusieurs capteurs montés parallèlement à l'axe longitudinal.

5. Assemblage tubulaire selon l'une quelconque des revendications 1 à 4, caractérisé en ce que le tube (11) est réalisé à partir d'un polymère thermoplastique extrudé, et que son traitement thermique entraîne une relaxation des contraintes résiduelles et provoque le déplacement de sa surface intérieure en direction du bouchon (12) pour s'y lier.

6. Procédé d'assemblage d'un tube creux allongé (11) avec un axe le long de sa longueur et des surfaces extérieures, une extrémité proximale et une extrémité distale (16) et un bouchon (12) avec une forme et une dimension en section transversale analogues à celles de la surface intérieure du tube (11) lorsque le bouchon (12) se trouve au moins partiellement à l'intérieur du tube (11) en utilisant un moule (18') formé et dimensionné pour se monter vers la surface extérieure du tube (11) et un moyen pour chauffer (19) associé avec le moule (18') ; caractérisé par les étapes suivantes consistant à :
- maintenir le tube (11) et le bouchon (12) de sorte qu'un mouvement relatif entre eux et le long de l'axe du tube (11) place le bouchon (12) au moins partiellement à l'intérieur du tube (11) ;
- positionner le moule (18') pour un mouvement relatif le long de l'axe pour une mise en place vers le tube (11) à l'intérieur duquel se trouve le bouchon (12) de façon à enfermer la surface extérieure du tube ;
- soumettre le tube (11) et le bouchon (12) à un traitement thermique pour modifier la température de manière suffisante pour relaxer des contraintes résiduelles et de façon à provoquer une liaison de la surface intérieure du tube (11) avec le bouchon (12), laquelle liaison forme un joint étanche au fluide qui ferme hermétiquement l'extrémité distale (16) du tube (11).

7. Procédé selon la revendication 6, comprenant l'étape consistant à dégager un fluide vers la jonction entre le bouchon (12) et le tube (11) avant de chauffer.

8. Dispositif (10) pour assembler un tube (11) et un bouchon (12) au moins partiellement inséré à l'intérieur de celui-ci, caractérisé par :
- un tube creux allongé (11) avec un axe le long de sa longueur, et avec des surfaces intérieure et extérieure, une extrémité proximale et une extrémité distale (16) ;
- un bouchon (12) avec une forme et une dimension en section transversale analogues à celles de la surface intérieure lorsque le bouchon (12) se trouve au moins partiellement à l'intérieur du tube (11), et le bouchon (12) et le tube (11) étant maintenus de sorte qu'un mouvement relatif le long de l'axe place le bouchon (12) au moins partiellement à l'intérieur du tube (11) ;
- un moule (18') positionné pour un mouvement relatif le long de l'axe pour une mise en place autour du tube (11) à l'intérieur duquel se trouve le bouchon (12), le moule étant formé et dimensionné pour se monter à la surface extérieure du tube (11) et l'enfermer ; et
- un moyen pour chauffer (19) associé au moule pour assurer un traitement thermique pour modifier de manière suffisante la température du tube (11) et du bouchon (12) pour relaxer des contraintes résiduelles et de façon à permettre une liaison entre la surface intérieure du tube (11) et le bouchon (12), laquelle liaison forme un joint étanche au fluide qui ferme hermétiquement l'extrémité distale (16) du tube (11).

9. Dispositif selon la revendication 8, caractérisé en ce que le tube (11) a une section transversale intérieure sensiblement circulaire et une dimension en section transversale plus importante que celle du bouchon (12) avant traitement thermique et le bouchon a une section transversale externe sensiblement circulaire de dimension analogue à celle de la section transversale intérieure du tube (11) après traitement thermique.

10. Dispositif selon la revendication 8 ou 9, caractérisé en ce que le bouchon (12) est réalisé en un polymère thermoplastique par un processus qui révèle à l'intérieur du polymère une mémoire du polymère thermoplastique non formé avant d'être moulé dans la forme en section transversale du bouchon (12) de sorte que des contraintes résiduelles subsistant dans le bouchon (12) sont relaxées en modifiant une température provoquant une dilatation de la dimension en section transversale du bouchon (12).
